(19) Europäisches Patentamt
European Patent Office
Office européen des brevets

(11) **EP 1 974 660 A1**

(12) **EUROPEAN PATENT APPLICATION**
published in accordance with Art. 158(3) EPC

(43) Date of publication:
01.10.2008 Bulletin 2008/40

(51) Int Cl.:
*A61B 5/00* (2006.01)   *A61B 6/00* (2006.01)
*G09G 3/20* (2006.01)   *G09G 3/36* (2006.01)
*G09G 5/00* (2006.01)   *G09G 5/02* (2006.01)

(21) Application number: 07706563.9

(22) Date of filing: 11.01.2007

(86) International application number:
PCT/JP2007/050216

(87) International publication number:
WO 2007/083556 (26.07.2007 Gazette 2007/30)

(84) Designated Contracting States:
AT BE BG CH CY CZ DE DK EE ES FI FR GB GR
HU IE IS IT LI LT LU LV MC NL PL PT RO SE SI
SK TR

(30) Priority: 19.01.2006 JP 2006011197

(71) Applicant: Konica Minolta Medical & Graphic, Inc.
Hino-shi,
Tokyo 191-8511 (JP)

(72) Inventor: NAKAZAWA, Masayuki
Tokyo 163-0512 (JP)

(74) Representative: Gille Hrabal Struck Neidlein Prop
Roos
Patentanwälte
Brucknerstrasse 20
40593 Düsseldorf (DE)

(54) **IMAGE DISPLAY, IMAGE DISPLAY METHOD, AND IMAGE DISPLAY PROGRAM**

(57)    An image display, image display method, and image display program for displaying a medical monochromatic image in the field of medical care with a color tone suitable to detect a lesion. The image display comprises a control section for distributing monochromatic image data into R data, G data, and B data to create display image data and a color display section for displaying a monochromatic image according to the display image data. The control section distributes the monochromatic image data into R data, G data, and B data within the range of the maximum luminance L0 of when at least an image is displayed to the luminance L1 which is one thirtieth in such a way that the following relations are satisfied: R data = KR · B data (0.5 < KR ≤ 1), D data = KG · B data (0.5 < KG ≤ 1), and 0.85 < KG/KR < 1.2 to create display image data.

FIG. 3

EP 1 974 660 A1

**Description**

**TECHNICAL FIELD**

The present invention relates to an image display apparatus, image display method and image display program, particularly to an image display apparatus, image display method and image display program which ensure that the so-called blue-based monochrome image used in the medical field is displayed in a color tone suitable for diagnosis.

**BACKGROUND ART**

The diagnostic image captured by a diagnostic apparatus for medical treatment such as an X-ray diagnostic apparatus including a CR (Computed Radiography), an MRI (Magnetic Resonance Imaging) diagnostic apparatus, and various types of CT (Computed Tomography) apparatuses, is normally recorded on a transparent image recording film such as an X-ray film and other film photosensitive materials, and is reproduced as an transparent image. The film with the diagnostic image reproduced thereon is set on the apparatus for observation under the name of schaukasten, and is observed with light emitted thereto from the back, whereby presence or absence of a lesion, for example, is examined.

The diagnostic and measuring apparatus for medical treatment of various types is connected with a CRT (Cathode Ray Tube) display or LCD (Liquid Crystal Display) as a monitor for observation of the image having been captured and measured. The image outputted to these monitors is used for verification and adjustment of the diagnostic image prior to diagnosis or film output, or for processing of the image.

When the image captured by the aforementioned diagnostic apparatus for medical treatment is reproduced on the film or when the image captured or measured by the diagnostic and measuring apparatus for medical treatment is reproduced on the film, the so-called blue-based monochrome film is normally used as a film in many cases.

In the meantime, the image to be displayed on the monitor is displayed on the white-based screen. This makes it difficult for a doctor observing both the film and monitor to intuitively adjust to both of the observation on the film and that on the monitor. The doctor accustomed to observe either of the images will find it difficult to observe the other image. This problem has been left unsolved in the conventional art.

To solve the aforementioned problem, a proposal has been made of an image display method for displaying the image wherein, for example, when the chromaticity of the displayed image is represented in terms of coordinates (x, y) on the CIE chromaticity coordinate, an image is displayed in such a way as to be located in the area enclosed by the coordinates (0.174, 0), (0.4, 0.4) and ($\alpha$, 0.4), where $\alpha$ indicates the coordinate x at the crossing point formed by the spectral locus and a straight line wherein the coordinate in the y-axis direction is 0.4 (see Patent Documents 1 and 2). This technique provides an image of blue-based color tone even when a monochrome image is displayed on the white-based monitor screen.

[Patent Document 1] Unexamined Japanese Patent Application Publication No. 2000-330530
[Patent Document 2] Unexamined Japanese Patent Application Publication No. 2001-34232

**DISCLOSURE OF THE INVENTION**

**PROBLEMS TO BE SOLVED BY THE INVENTION**

However, the present inventors have found out that, even if the chromaticity of the image displayed on the monitor screen is located within the aforementioned range, an image preferably used for diagnosis cannot always be obtained, for example, due to the excessive intensity of the blue color tone.

This will cause difficulties in adjustment to both of the color tone of the film and that of the monitor screen, and hence will bring discomfort. Not only that, this is connected to the problem of detectability of a lesion such that an observer including a doctor cannot identify the lesion denoted by the image. This may lead to a serious problem that causes misdiagnosis.

Further, the color tone varies according to the type of the film. It has been found out that there is a great variation in the color tone according to the type of the film, and the color tone familiar with use or the tone color of personal preference varies according to each doctor.

An object of the present invention is to solve the aforementioned problems and to provide an image display apparatus and image display method which ensure that a monochrome image for medical use in the field of medical treatment is displayed in the color tone suitable for detection of a lesion.

**MEANS TO SOLVE THE PROBLEMS**

To solve the above problems, an image display apparatus described in Claim 1 comprises: a control section generating image data to be displayed by assigning data of a monochrome image to R, G, and B; and a color display section which is capable of displaying the monochrome image based on the image data to be displayed, wherein the control section generates the image data to be displayed by assigning the data of the monochrome image to R, G, and B so as to satisfy a relationship between following formulas (1) and (2) within at least a range between a maximum luminance L0 of an image to be displayed and a luminance L1 which is one thirtieth of the luminance L0:

$$\text{Data-R} = KR \cdot \text{data-B} \; (0.5 < KR \leq 1) \qquad \text{Formula (1)}$$

$$\text{Data-G} = KG \cdot \text{data-B} \; (0.5 < KG \leq 1) \qquad \text{Formula (2)}$$

where coefficients KR and KG in the formulas satisfy a condition of a following formula (3):

$$0.85 < KG/KR < 1.2. \qquad \text{Formula (3)}$$

Further, as for an invention described in Claim 2, according to the image display apparatus of Claim 1, wherein the coefficients KR and KG satisfy a following relationship:

$$1.0 < KG/KR < 1.15.$$

An invention described in Claim 3, according to the image display apparatus of Claim 1 or 2, comprises: a color tone inputting means of inputting a desired color tone, wherein the control section generates the image data to be displayed by assigning the data of the monochrome image to R, G, and B according to the color tone inputted by the color tone inputting means.

An invention described in Claim 4, according to the image display apparatus of Claim 1 or 2, comprises: an observer identification code inputting means of inputting an observer identification code which can identify an observer; and a storage section storing the observer identification code and a color tone desired by each observer with associated with each other and, wherein the control section generates the image data to be displayed by assigning the data of the monochrome image to R, G, and B according to a color tone desired by an observer who is associated with the observer identification code inputted by the observer identification code inputting means.

An image display method for displaying a monochrome image on a color display section based on an image data to be displayed generated by assigning data of the monochrome image to R, G, and B, comprises: generating the image data to be displayed by assigning the data of the monochrome image to R, G, and B so as to satisfy a relationship of following formulas (1) and (2) within at least a range between a maximum luminance L0 of an image to be displayed and a luminance L1 which is one thirtieth of the luminance L0:

$$\text{Data-R} = KR \cdot \text{data-B} \; (0.5 < KR \leq 1) \qquad \text{Formula (1)}$$

$$\text{Data-G} = KG \cdot \text{data-B} \; (0.5 < KG \leq 1) \qquad \text{Formula (2)}$$

where coefficients KR and KG in the formulas satisfy a following condition of a following formula (3):

$$0.85 < KG/KR < 1.2. \qquad \text{Formula (3)}$$

As for an invention described in Claim 6, according to the image display method of Claim 5, the coefficients KR and KG satisfy a following relationship:

$$1.0 < KG/KR < 1.15.$$

Further, as for an invention described in Claim 7, according to the image display method of Claim 5 or 6, when a desired color tone is inputted, the image data to be displayed is generated by assigning the data of the monochrome image to R, G, and B according to the inputted color tone.

As for an invention described in Claim 8, according to the image display method of Claim 5 or 6, when an observer identification code which can identify the observer is inputted, the image data to be displayed is generated by assigning the data of the monochrome image to R, G, and B according to a desired color tone associated with each observer.

An invention described in Claim 9 causes a computer to carry out the method of any one of Claims 5 to 8.

**EFFECT OF THE INVENTION**

According to the inventions described in Claims 1, 5 and 9, an image data to be displayed is generated from the data of the monochrome image, wherein this image data to be displayed is similar to the color tone of the blue-based film with an appropriate level of chromaticity. These inventions allow the image to be displayed on a color display section such as a liquid crystal panel in the form (property) of display suitable for diagnosis, with the minimum sense of discomfort between the color tone of the film and that of the monitor screen for the doctor as an observer. Further, these inventions allow the doctor or other observer to have a sufficient identification of a lesion on the monitor screen, thereby ensuring adequate detection of a lesion.

Particularly, the range between the maximum luminance L0 of an image to be displayed and the luminance L1 of about one 30th thereof corresponds to the range of density (range of luminance) wherein human eyes are particularly sensitive to the difference of the color tone. Thus, more remarkable effects can be expected by adjusting the color tone in this range of density (range of luminance).

According to the inventions described in Claims 2 and 6, an image data to be displayed is generated from the data of the monochrome image, wherein this image data to be displayed is similar to the color tone of the blue-based film with an appropriate level of chromaticity. These inventions allow the image to be displayed on a color display section in the form (property) of display suitable for diagnosis, with the minimum sense of discomfort between the color tone of the film and that of the monitor screen for the doctor as an observer. Further, these inventions allow the doctor or other observer to have a sufficient identification of a lesion on the monitor screen, thereby ensuring adequate detection of a lesion.

According to the inventions described in the Claims 3 and 7, the color tone varies according to the type of the film. Even when the color tone familiar with use or the tone color of personal preference varies according to each doctor as an observer, a desired color tone is inputted and an image data to be displayed is generated based thereon. This feature ensures that the image of the color tone conforming to the taste of the observer is displayed on the monitor screen.

According to the inventions described in the Claims 4 and 8, the color tone varies according to the type of the film. Even when the color tone familiar with use or the tone color of personal preference varies according to each doctor as an observer, the observer identification code corresponding to a desired color tone is inputted and an image data to be displayed of the color tone desired by each observer is generated based thereon. This feature ensures that the image of the color tone conforming to the taste of the observer is displayed on the monitor screen.

The invention described in Claim 9, causes a computer to carry out the image display method described in any one of the Claims 5 through 8.

**BRIEF DESCRIPTION OF THE DRAWINGS**

Fig. 1 is a block diagram schematically representing a first embodiment of the image display apparatus of the present invention;

Fig. 2 is a block diagram schematically representing the control section of the image display apparatus illustrated in Fig. 1;

Fig. 3 is a drawing showing the preferable area for the chromaticity of the display image data in the present embodiment;

Fig. 4 is a drawing showing the data representing the color tones of various types of films;

Fig. 5 is a drawing showing the example of assigning the RGB data to the frame data;

Fig. 6 is a timing chart showing the processing of an image in the present embodiment;

Fig. 7 is a block diagram schematically representing the structure of a variation of the image display apparatus shown in Fig. 1; and

Fig. 8 is a block diagram schematically representing the structure of a variation of the image display apparatus shown in Fig. 1.

## EXPLANATION OF NOTATION

1. Image display apparatus
2. Liquid crystal panel
3. Liquid crystal display drive section
4. Backlight
5. Control section
6. Interface
7. Image supply apparatus
9. Data processing section
11. Frame switching section

## BEST MODE FOR CARRYING OUT THE INVENTION

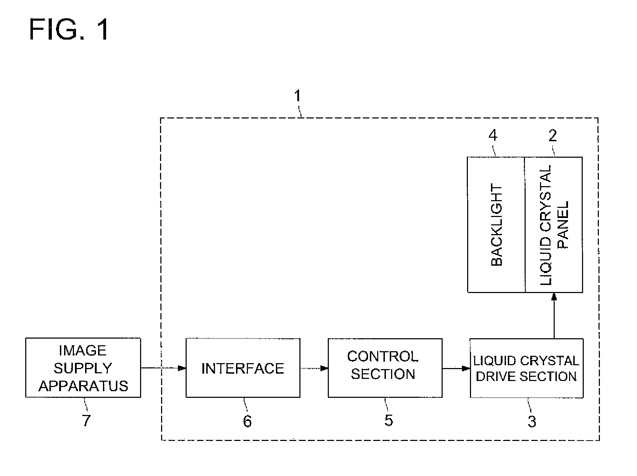

The following describes an embodiment of the present invention with reference to Figs. 1 through 6, without the present invention being restricted to the illustrated examples.

Fig. 1 is a block diagram schematically representing a first embodiment of the image display apparatus of the present invention. In this embodiment, an image display apparatus 1 is a monitor of the diagnostic apparatus for medical treatment, and is provided with a liquid crystal panel (LCD (Liquid Crystal Display)) 2 as the color display section for displaying an image through a liquid crystal, and a liquid crystal drive section 3 as a display drive section for driving the liquid crystal panel, as shown in Fig. 1.

There is no restriction to the type of the liquid crystal panels 2 applicable to the present embodiment. The liquid crystal panel 2 can be driven by the liquid crystal drive section 3 according to various forms of drive methods such as TN (Twisted Nematic), ST (Super Twisted Nematic) and MVA (Multi-domain Vertical Alignment) drive methods.

The image display apparatus 1 is provided with a backlight 4 for emitting light to the liquid crystal panel 2 from the non-observer side. The backlight 4 is required only to emit enough amount of light to the liquid crystal panel 2. For example, it is possible to employ an LED, cold cathode fluorescent tube, hot cathode fluorescent tube, or other light emitting devices. It is preferred to have a maximum display luminance of 200 through 5000 $cd/m^2$ so as to ensure preferable use in a monitor for medical treatment.

Further, the image display apparatus 1 is provided, for example, with a CPU (Central Processing Unit), a ROM (Read On Memory) for storing various types of control programs, and a RAM (Random Access Memory) for temporary storage of image data (these devices not illustrated). It is also provided with a control section 5 for controlling the aforementioned liquid crystal drive section 3, and an interface (I/F) 6 for connection with the control section 5 and external apparatus.

In the image display apparatus 1 according to the present embodiment, various types of image supply apparatuses 7 as the sources for supplying the monochrome image data for diagnosis are connected with the control section 5 of the image display apparatus 1 through the interface 6. The image supply apparatuses 7 connected to the image display apparatus 1 are exemplified by diagnostic apparatuses for medical treatment, including an X-ray diagnostic apparatus such as CR (Computed Radiograph), MRI (Magnetic Resonance Imaging) diagnostic apparatus and various types of CT (Computed Tomography) apparatuses. The image supply apparatus 7 is not restricted to the illustrated ones. The image storage apparatus such as an image server connected to the network can be used as such.

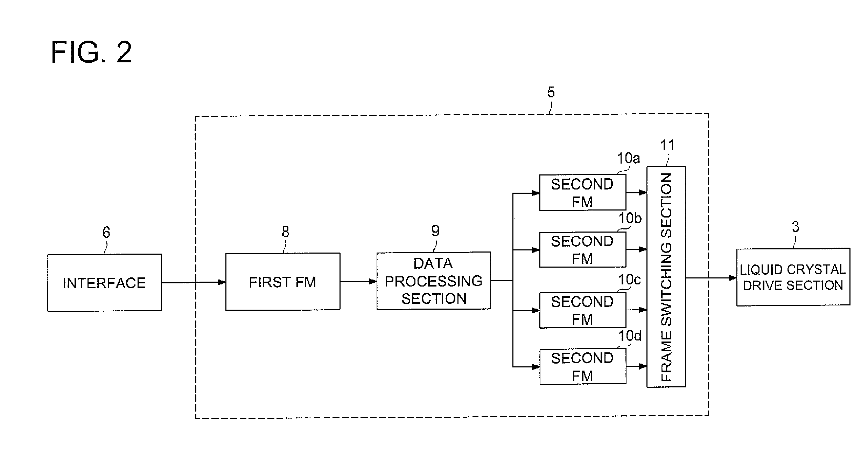

The following describes the control section 5 of the present embodiment with reference to Fig. 2.

As shown in Fig. 2, the control section 5 includes: a first frame memory (Flame Memory: "FM" in Fig. 2) 8 for storing the image data inputted from the image supply apparatus 7 through the interface 6; a data processing section 9 for converting the image data stored in the first frame memory 8 to the image data to be displayed (RGB data) by assigning the stored image data to R, G, and B, and for assigning this image data to be displayed (RGB data) to the frame data for FRC (Flame Rate Control: time sharing) display; four second frame memories (FM) 10a, 10b, 10c and 10d for storing the frame data assigned by the data processing section 9; and a frame switching section 11 for sequential switching of the image data to be displayed stored in the second frame memories 10a, 10b, 10c and 10d at the time of FRC display, thereby outputting the data the liquid crystal drive section 3.

When a display apparatus is used to display the image data having a greater gradation resolution (number of bits), an image data group of a lower number of bits is generated from the image data having a greater number of bits in the number corresponding to the difference in the number of bits of the both, and this image data group is sequentially displayed, whereby gradation representation equivalent to the greater number of bits is provided by an image display apparatus of a lower number of bits. This procedure is defined as the FRC display.

To put it more specifically, the data processing section 9 generates the image data group of a lower number of bits having the number of frames of $2^n$ wherein the difference in the number of bits is assumed as "n", and this image data of a lower number of bits is sequentially displayed, whereby gradation representation equivalent to 10-bit gradation resolution is provided using four frames of image with an 8-bit gradation resolution. This procedure permits display an artifact-free image, even if high luminance is higher.

The following describes the embodiment wherein the image data having a 10-bit gradation resolution is displayed at an 8-bit gradation resolution. In the present embodiment, the monochrome image data having a 10-bit gradation resolution is supplied to the control section 5 from the image supply apparatus 7 through the interface 6. Further, the image display apparatus 1 displays an image at an 8-bit gradation resolution. Thus, after having been stored in the first frame memory 8, the 10-bit monochrome image data is read out into the data processing section 9, and is converted into the 8-bit display image data.

In other words, in the present embodiment, the data processing section 9 assigns 1024 pieces of data ranging from 0 through 1023 as the original 10-bit monochrome image data (original image data) to each of the R, G and B so as to satisfy the following, whereby the data is converted into 8-bit display image data:

```
Data-B = "original image data"/4 (discard all digits to the right of decimal point)

    Data-R = KR x data-B (discard all digits to the right

of decimal point)

    Data-G = KG x data-B (discard all digits to the right

of decimal point)   ... Formula (1)
```

FRC display is not used at the time of display in the 8-bit gradation resolution mode. Thus, it is sufficient only if the same display image data is stored in each of the frame memories 10a, 10b, 10c and 10d. In this case, the value is assumed to satisfy $0.5 < KR \leq 1.0$, and $0.5 < KG \leq 1.0$.

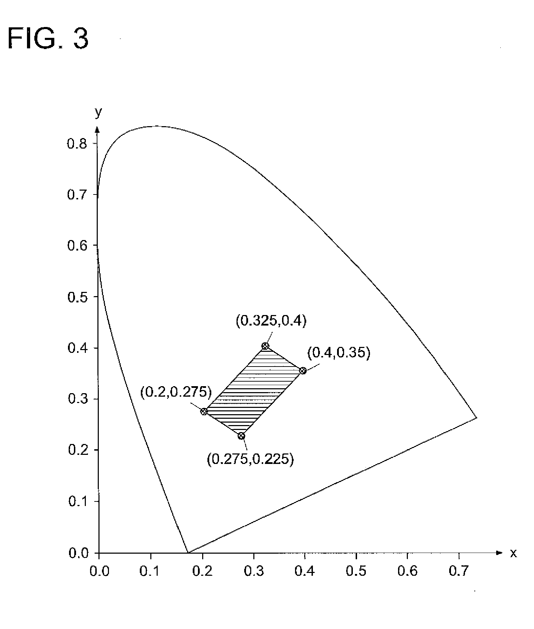

Fig. 3 shows the color tone when the images of various types of blue-based monochrome films are displayed in the schaukasten mode. The films used include three films based on photothermal silver halide method, one film based on thermal silver halide method and one film based on heat-sensitive method wherein a microcapsule is impregnated with pigment. Three fluorescent lamps having different color tones (white: color temperature of 4200K, neutral white color: color temperature of 5000K, and daylight color: color temperature of 6500K) were used as schaukasten light sources. The coordinate of Fig. 3 indicates the coordinate (x, y) of CIE chromaticity. Blue is deeper as one goes closer to the left bottom (wherein values x and y are smaller).

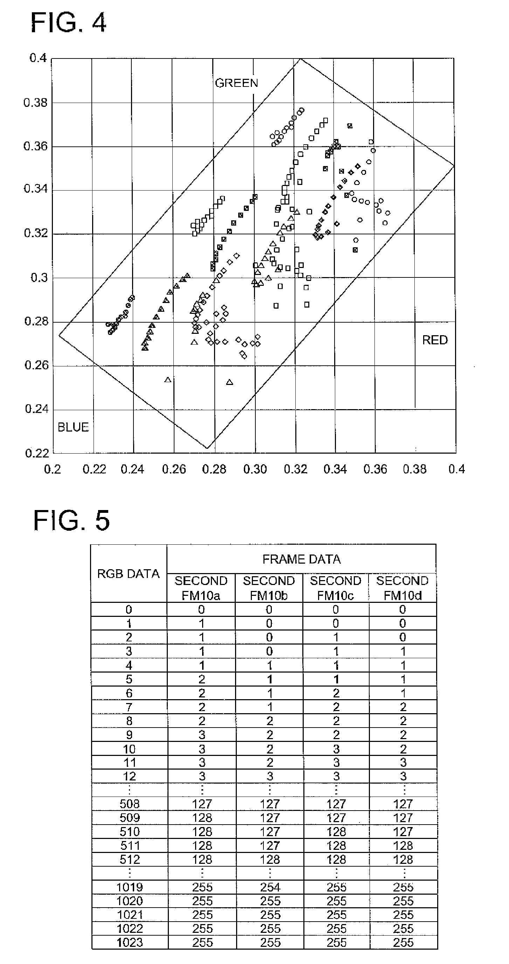

Fig. 4 is a partially enlarged view of Fig, 3. Each plot indicates the actually measured values of the chromaticity of each film, each light source and each density in the film.

In the Unexamined Japanese Patent Application Publications Nos. 2000-330530 and 2001-34232, the chromaticity of the displayed image is located within the range enclosed by the coordinates (0.174, 0), (0.4, 0.4), (α, 0.4) on the CIE chromaticity coordinate (wherein α indicates the coordinate x at the crossing point formed by the spectral locus and a straight line wherein the coordinate in the y-axis direction is 0.4).

However, the present inventors have found out that, when various types of blue-based monochrome films are observed by the schaukasten, image chromaticity is concentrated in the range considerably narrower than that indicated in the Unexamined Japanese Patent Application Publications Nos. 2000-330530 and 2001-34232, as shown in Fig. 4. The chromaticity of Fig. 4 denotes the results of actual measurement of the chromaticity in each film type, each light source and each film density.

When consideration is given to the chromaticity measurement error, variations and chronological changes in the film color tone, variations and chronological changes and chromaticity in the light source of the schaukasten, and resolution of human eyes, an allowance of about ±0.01 through 0.02 is preferably given to the measurement data of Figs. 3 and 4 for both x and y. Thus, when the chromaticity of the image displayed in at least the range between the maximum luminance L0 of an image to be displayed and the luminance L1 of about one 30th thereof is shown in terms of the coordinates (x, y) on the CIE chromaticity coordinate, the chromaticity preferably used for reproduction of the color tone at the time of observation of the film should be located in the range enclosed by the coordinates (0.2, 0.275), (0.275, 0.225), (0.325, 0.4) and (0.4, 0.35).

Accordingly, in order to attain this range of chromaticity in the image display apparatus 1 according to the present embodiment, monochrome image data (original image data) is assigned to each of the RGB data item using the aforementioned formula (1), with setting the coefficient KR and coefficient KG to values capable of meeting 0.5 < KR ≤ 1.0, and 0.5 < KG ≤ 1.0, and further 0.85 < KG/KR < 1.2. It should be noted that KG/KR satisfies 1.0 < KG/KR < 1.15 more preferably.

The range between the maximum luminance L0 of an image to be displayed and the luminance L1 of one 30th thereof is equivalent to about 0.2 through about 1.7 in terms of the density range when observing the film. Human eyes are particularly sensitive to the difference in color tone in this range of density (range of luminance). Thus, strict adjustment of the color tone is preferred "within at least the range between the maximum luminance L0 of an image to be displayed and the luminance L1 of about one 30th thereof".

In the image display apparatus 1 according to the present invention, as described above, the image data to be displayed assigned to the R, G, and B by the data processing section 9 is supplied from the control section 5 to the liquid crystal drive section 3 of the liquid crystal panel 2, whereby the monochrome image can be displayed on the liquid crystal panel 2 in such a way that the blue tint will change according to the density of the image.

The aforementioned description uses an example of the case wherein the image data containing the 10-bit gradation resolution supplied from the image supply apparatus 7 is displayed at an 8-bit gradation resolution in the image display apparatus 1. As will be descried below, it is also possible to make such arrangements that the image data having a 10-bit gradation resolution supplied from the image supply apparatus 7 is displayed at a 10-bit gradation resolution in the image display apparatus 1 using the aforementioned FRC display function.

In this case, the data processing section 9 uses the aforementioned FRC display to operate in such a way that the image data having a 10-bit gradation resolution supplied from the image supply apparatus 7 is displayed in terms of four frames of image having 8-bit gradation resolution.

For example, the following steps are taken to provide a 10-bit display of the aforementioned original monochrome image data (original image data) in an 8 bits x 4-frame FRC display mode.

In the data processing section 9, 1024 data items ranging from 0 to 1023 as the original 10-bit monochrome image data (original image data) is assigned to the R, G, and B so as to satisfy the following, whereby the data is converted into 8-bit display image data:

```
Data-B = "original image data"

Data-R = KR x data-B (discard all digits to the right
of decimal point)

Data G = KG x data-B (discard all digits to the right
of decimal point) ... Formula (2)
```

In this case, coefficient KR and coefficient KG are set the values capable of meeting 0.5 < KR ≤ 1.0, 0.5 < KG ≤ 1.0, and 0.85 < KG/KR < 1.2.

In the data processing section 9, as shown in the aforementioned formula (2), the image data to be displayed (RGB data) assigned to R, G, and B is assigned to the data (frame data) of a plurality of FRC frames (time sharing frames) to conduct FRC display.

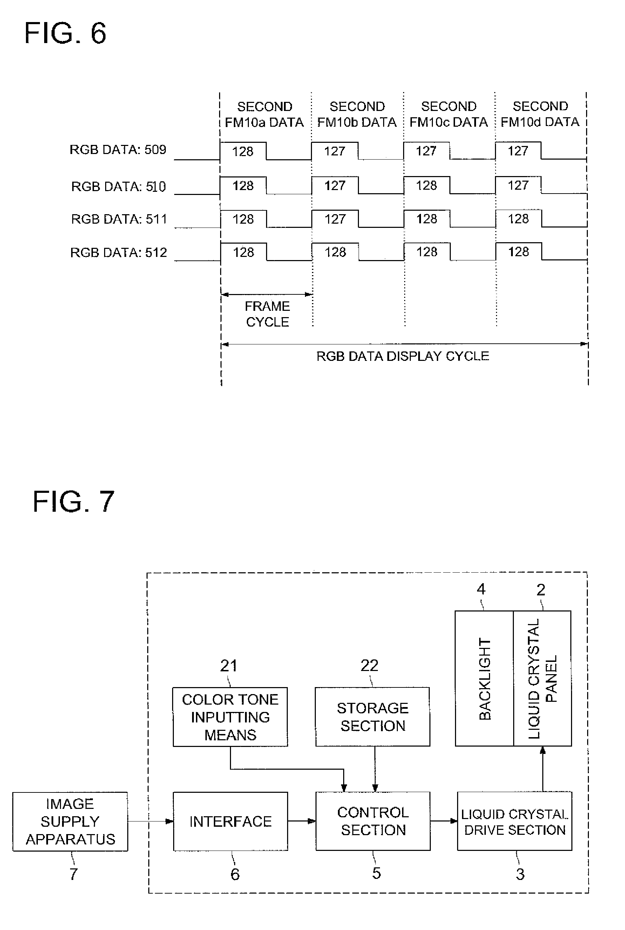

Fig. 5 shows an example of the frame data assigned to each of the second frame memories 10a, 10b, 10c and 10d when there are four second frame memories ("FM" in Figs. 5 and 6) 10a, 10b, 10c and 10d for FRC display.

As described above, the 10-bit image display can be achieved in an 8-bit x 4-frame time shared display mode. To be more specific, as shown in Fig. 6, the 10-bit display image data (RGB data) "509", for example, can be represented in terms of the four frames ("128, 127, 127, 127") of 8-bit image data. Similarly, the 10-bit display image data (RGB data) "510" can be represented in terms of the four frames ("128, 127, 128, 127") of 8-bit image data, the display image data (RGB data) "511" can be represented in terms of the four frames ("128, 127, 128, 128") of 8-bit image data, the display image data (RGB data) "512" can be represented in terms of the four frames ("128, 128, 128, 128") of 8-bit image data.

The following describes the image display method of the present invention.

When the monochrome image data having a 10-bit gradation resolution has been inputted from the image supply apparatus 7 through the interface 6, the image data is stored in the first frame memory 8.

When an image is displayed in the 8-bit gradation resolution mode in the image display apparatus 1, the data processing section 9 assigns the monochrome image data stored in the first frame memory 8 to R, G, and B according to the aforementioned Formula (1) and generates the image data to be displayed. Further, when an image is displayed in the 10-bit gradation resolution mode also in the image display apparatus 1, the data processing section 9 assigns the data to R, G, and B according to the aforementioned Formula (2) and generates the image data to be displayed.

Further, the data processing section 9 assigns the image data to be displayed (RGB data) having been generated in this manner, as frame data to each of the frame memories 10a, 10b, 10c and 10d, as shown in Fig. 5. The frame data having been stored in the frame memories 10a, 10b, 10c and 10d is sequentially outputted to the liquid crystal drive section 3 from the control section 5 as the frame switching section 11 switches among frame memories 10a, 10b, 10c and 10d as required. The liquid crystal drive section 3 drives the liquid crystal panel 2 based on the frame data outputted from the control section 5, whereby an image of the color tone similar to that of the blue-based monochrome film is displayed on the liquid crystal panel 2 of the image display apparatus 1.

As described above, according to the present embodiment, the image data to be displayed which have an appropriate chromaticity similar to the color tone of the blue-based film is generated from the monochrome image data inputted from various types of image supply apparatuses. Thus, the image is displayed on the liquid crystal panel 2 in the form (property) of display suitable for at least diagnosis, with a minimized sense of discomfort between the color tone of the film and that of monitor screen for the doctor as an observer.

The chromaticity of the image displayed in this manner is adjusted to an appropriate level, whereby sufficient identification of a lesion indicated by the image can be achieved, and hence, the doctor or other observer can adequately detect the lesion on the monitor screen.

In the present embodiment, on the assumption that a certain color tone suitable for displaying the image on the blue-based monochrome film and the data assigning ratio for achieving the same are preset on the image display apparatus 1, the data processing section 9 assigns the monochrome image data to R, G, and B so as to provide a predetermined color tone, and generates the display image data. However, the color tone need not be preset.

For example, as shown in Fig. 7, the image display apparatus can be provided with a color tone inputting means 21 that allows an observer (e.g., doctor) to set a desired color tone, and a storage section 22 that stores, the ratio of assigning the monochrome image data to R, G, and B with associated with each other in order to implement each color tone. In this case, for example, the storage section 22 can be configured to store the values of the KR and KG corresponding to the input signals that are inputted from the color tone inputting means 21 as a table (LUT for Look-Up Table, not illustrated). In this case, it is possible to make such arrangements that the color tone inputting means 21 displays a plurality of film type names, and the observer selects a desired film type name from among them, whereby the color tone is inputted. It is also possible to make such arrangements that the color tone inputting means 21 displays a plurality of types of color tones, and the observer selects a desired color tone from among them. When a desired color tone has been inputted from the color tone inputting means 21, the control section 5 reads out the table corresponding to this color tone from the storage section 22. Based on this information, the data processing section 9 distributes the data to generate the image data to be displayed.

As described above, when the structure is so designed as to allow the observer to input a desired color tone, the observer can change the color tone of the image displayed on the liquid crystal display section 2 in conformity to the type of the film having used with the medical facilities, to the color tone of the film having been accustomed thereto, or to the color tone of his personal preference. Thus, this structure provides an image characterized by a minimized sense of discomfort, and hence, ensures adequate diagnosis.

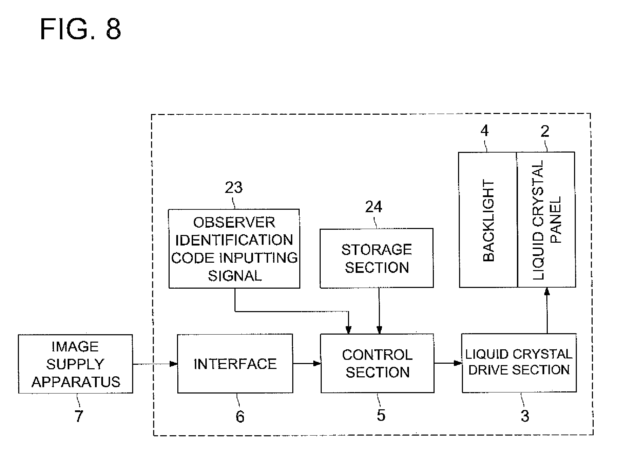

Further, when provided with an observer identification code such as observer ID that identifies each observer (e.g., doctor), the image display apparatus may include, for example, an observer identification code inputting means 23 for the observer (e.g., doctor) to input the observer identification code, and a storage section 24, as shown in Fig. 8. In this case, the color tone conforming to the personal preference of the observer is stored in the storage section 24 in advance, in the form associated with the observer identification code. When the observer identification code has been inputted to the control section 5 from the observer identification code inputting means 23, the control section 5 reads the color tone associated with the observer identification code having been inputted, from the storage section 24, and the data processing means 9 automatically selects this color tone. Thus, the data is assigned so as to get this color tone, whereby the display image data is generated.

As described above, when the observer inputs the observer identification code, a desired color tone is automatically selected. If this structure is used, the image is displayed on the liquid crystal display section 2 in conformity to the type of the film having used with the medical facilities, to the color tone of the film having been accustomed thereto, or to the color tone of his personal preference. Since the color tone familiar with use or the tone color of personal preference varies according to each doctor, it is a great benefit when the color tone can be changed for each observer by inputting the observer identification code without having to perform particular setup. Thus, this structure easily provides an image characterized by a minimized sense of discomfort, and hence, ensures adequate diagnosis.

In Figs. 7 and 8, the above description refers to the case wherein the color tone inputting means 21 or observer identification code inputting means 23 and storage sections 22 and 24 are provided integrally inside the image display apparatus equipped with the liquid crystal panel 2. It is also possible to arrange such a configuration that the whole or part thereof may be provided on the external apparatus such as an image supply apparatus.

It goes without saying that the present invention can be embodied in a great number of variations with appropriate modification or additions, without being restricted to the aforementioned embodiment.

**EXAMPLES**

The following specifically describes the image display apparatus and image display method of the present invention with reference to Examples. It is to be expressly understood, however, that the present invention is not restricted thereto.

[Example 1]

In this Example, a 3-megapixel color liquid crystal monitor (FA-2090) made by Eizo Nanao Corporation was used as the image display apparatus 1 illustrated in Fig. 1. The data on the image of the front of the 12-bit pectoral region captured by the REGIUS MODEL 190 manufactured by Konica Minolta Medical & Graphic, Inc. was used as the monochrome image data. Further, the luminance and chromaticity were measured at a viewing angle of 2° using the luminance meter (LS-100) manufactured by Konica Minolta Sensing, Inc.

In the present Example, 4096 data items from 0 to 4095 of the original monochrome image data (original image data) were assigned to R, G, and B so as to satisfy the following, where KR = 0.95 and KG = 0.95:

```
Data-B = "original image data"/4 (discard all digits to the right of decimal point)

Data-R = KR x data-B (discard all digits to the right

of decimal point)

Data G = KG x data-B (discard all digits to the right

of decimal point) ... Formula (3)
```

The image data to be displayed (RGB data) assigned as shown in Formula (3) is distributed to each of four frame memories according to the distribution shown in Fig. 5, and the four-split frame data is generated. Thus, gradation representation corresponding to the 10-bit gradation resolution was performed in the image display apparatus using the FRC display function similar to that of the aforementioned embodiment.

[Example 2]

An image was displayed in the same way as Example 1, except that KR = 0.75 and KG = 0.85.

[Example 3]

An image was displayed in the same way as Example 1, except that KR = 0.60 and KG = 0.58.

[Comparative example 1]

An image was displayed in the same way as Example 1, except that KR = 0.40 and KG = 0.40.

[Comparative example 2]

An image was displayed in the same way as Example 1, except that KR = 0.45 and KG = 1.0.

[Comparative example 3]

An image was displayed in the same way as Example 1, except that KR = 1.0 and KG = 0.45.
Table 1 shows the values KR and KG of the Examples 1 through 3 and comparative examples 1 through 3. Further, within the range between the maximum luminance L0 of an image to be displayed and the luminance L1 of about one 30th thereof, the result of measuring the chromaticity of the displayed image (x, y on the CIE chromaticity coordinate) was also shown. The chromaticity was almost constant without fluctuating according to the display luminance, and was kept within the range of $\pm 0.01$ for both x and y.

Table 1

| | KR | Condition 1 | KG | Condition 2 | KG/KR | Condition 3 | Chromaticity coordinate x | Chromaticity coordinate y | Condition 4 |
|---|---|---|---|---|---|---|---|---|---|
| Example 1 | 0.95 | O | 0.95 | O | 1.00 | O | 0.330±0.01 | 0.340±0.01 | O |
| Example 2 | 0.75 | O | 0.8 | O | 1.07 | O | 0.285±0.01 | 0.315±0.01 | O |
| Example 3 | 0.6 | O | 0.58 | O | 0.97 | O | 0.265±0.01 | 0.265±0.01 | O |
| Comparative example 1 | 0.4 | X | 0.4 | X | 1.00 | O | 0.220±0.01 | 0.215±0.01 | X |
| Comparative example 2 | 0.45 | X | 1 | O | 2.22 | X | 0.235±0.01 | 0.365±0.01 | X |
| Comparative 1 example 3 | | O | 0.45 | X | 0.45 | X | 0.360±0.01 | 0.260±0.01 | X |

The following describes the conditions 1 through 4 in Table 1:

Condition 1: $0.5 < KR \leq 1$

Condition 2: $0.5 < KG \leq 1$

Condition 3: $0.85 < KG/KR < 1.2$

Condition 4: The measurement of the chromaticity of the displayed image is located within the area enclosed by coordinates (0.2, 0.275), (0.275, 0.225), (0.325, 0.4) and (0.4, 0.35).

In the Condition column of Table 1, a circle (O) is given if these conditions are met, and a cross (X) is given if not.
When the coefficient KR and coefficient KG meet the Conditions 1 through 3 in Table 1, viz., if they meet $0.5 < KR \leq 1$, $0.5 < KG \leq 1$, $0.85 < KG/KR < 1.2$, the chromaticity of the displayed image is confirmed to be located within the area enclosed by the coordinates (0.2, 0.275), (0.275, 0.225), (0.325, 0.4) and (0.4, 0.35).
Table 2 shows the result of evaluation by four doctors A, B, C and D visually viewing the color tone of each image in each of the Examples and comparative examples, wherein the results are rated on a scale of 1 through 5. At the time of visual evaluation, setting was made so that the luminance of the monitor (liquid crystal panel) would be 1.0 through 200 cd/m$^2$. The illuminometer (LX-1330) manufactured by Custom K.K. was used for measurement, and the environment for observation was set to about 20 1x. This is the luminance that does not affect the minimum luminance of the monitor.

Table 2

| | Doctor A | Doctor B | Doctor C | Doctor D | Total score | Remarks |
|---|---|---|---|---|---|---|
| Example 1 | 4 | 3 | 4 | 5 | 16 | Color tone similar to that of the film |
| Example 2 | 5 | 4 | 5 | 4 | 18 | Color tone similar to that of the film |
| Example 3 | 4 | 5 | 4 | 3 | 16 | Color tone similar to that of the film |
| Comp. 1 | 2 | 3 | 2 | 1 | 7 | Excessively bluish tone |
| Comp. 2 | 1 | 2 | 1 | 1 | 6 | Excessively greenish tone |
| Comp. 3 | 1 | 1 | 1 | 2 | 5 | Excessively reddish tone |
| Comp.; Comparative example | | | | | | |

In Table 2, an image having a more preferable color tone receives a higher score. Comparison was made using the total scores of four doctors. It has been revealed that the Example meeting all the Conditions 1 through 4 clearly receives a higher score than the comparative example, and the color tone is more preferable. Further, of these Examples, Example 2 meeting $1.0 < KG/KR < 1.15$ was placed in the top rank, which is preferable. The Remarks column includes the comments by the doctors. In the Examples 1 through 3 gaining higher scores, all four doctors felt the color tone similar to that of the film. By contrast, in the comparative examples 1 through 3 gaining poor scores, they felt excessively bluish tone in the comparative example 1, excessively greenish tone in the comparative example 2, and excessively reddish tone in the comparative example 3.
Table 3 shows the result of evaluation by four doctors A, B, C and D visually checking the detectability of the shadow of a tumor in the lung field in each image in the Examples and comparative examples, wherein the results are rated on a scale of 1 through 5. At the time of visual evaluation, setting was made so that the luminance of the monitor (liquid crystal panel) would be 1.0 through 200 cd/m$^2$, similarly to the case of visual observation shown in Table 2. The environment for observation was set to about 20 lx. This is the luminance that does not affect the minimum luminance of the monitor.

Table 3

| | Doctor A | Doctor B | Doctor C | Doctor D | Total score | Remarks |
|---|---|---|---|---|---|---|
| Example 1 | 5 | 4 | 4 | 3 | 16 | No diagnostic problem |
| Example 2 | 4 | 5 | 5 | 4 | 18 | No diagnostic problem |
| Example 3 | 3 | 4 | 4 | 4 | 15 | No diagnostic problem |

(continued)

|  | Doctor A | Doctor B | Doctor C | Doctor D | Total score | Remarks |
|---|---|---|---|---|---|---|
| Comp. 1 | 1 | 2 | 2 | 1 | 7 | Possible misdiagnosis |
| Comp. 2 | 2 | 1 | 1 | 1 | 6 | Possible misdiagnosis |
| Comp. 3 | 1 | 1 | 1 | 1 | 5 | possible misdiagnosis |
| Comp.; Comparative example | | | | | | |

In Table 3, an image having a higher detectability of the shadow of a tumor in the lung field (easier to find the shadow of a tumor) receives a higher score. Comparison was made using the total scores of four doctors. It has been revealed that the Example meeting all the Conditions 1 through 4 clearly receives a higher score than the comparative example, and the detectability of the tumor shadow is higher. Further, of these Examples, it can be shown that Example 2 meeting 1.0 < KG/KR < 1.15 was placed in the top rank which is preferable. The Remarks column includes the comments by the doctors. In the Examples 1 through 3 gaining higher scores, all four doctors evaluated the detectability as having no diagnostic problem. By contrast, in the comparative examples 1 through 3 gaining poor scores, they felt that misdiagnosis might occur.

Example 2 registered the best score in terms of the overall evaluation by the doctors A through D. The different Example gained the highest score according to different doctor, and this has verified that there are differences in preference according to individual doctors. Thus, as described above, the image display apparatus is preferably provided with a color tone inputting means 21 for the observer (e.g., doctor) to input a desired color tone, and a storage section 22 for storing, in an associated form, the ratio of assigning the monochrome image data to R, G, and B in order to implement each color tone, whereby the observer is allowed to select a desired color tone.

Further, when the observer identification code such as an observer ID capable of identifying the individual for each observer (e.g., doctor) is provided, as described above, the image display apparatus is preferably provided with an observer identification code inputting means 23 for the observer (e.g., doctor) to input an observer identification code, and a storage section 24 for storing the color tone preferable to the observer with associated with the observer identification code in advance, in such a way that a desired color tone is automatically selected when the observer has inputted the observer identification code.

**Claims**

1. An image display apparatus comprising:

   a control section generating image data to be displayed by assigning data of a monochrome image to R, G, and B; and
   a color display section which is capable of displaying the monochrome image based on the image data to be displayed,

   wherein the control section generates the image data to be displayed by assigning the data of the monochrome image to R, G, and B so as to satisfy a relationship between following formulas (1) and (2) within at least a range between a maximum luminance L0 of an image to be displayed and a luminance L1 which is one thirtieth of the luminance L0:

   $$\text{Data-R} = \text{KR} \cdot \text{data-B} \quad (0.5 < \text{KR} \leq 1) \qquad \text{Formula (1)}$$

   $$\text{Data-G} = \text{KG} \cdot \text{data-B} \quad (0.5 < \text{KG} \leq 1) \qquad \text{Formula (2)}$$

   where coefficients KR and KG in the formulas satisfy a condition of a following formula 3:

   $$0.85 < \text{KG/KR} < 1.2. \qquad \text{Formula (3)}$$

**2.** The image display apparatus of Claim 1,
wherein the coefficients KR and KG satisfy a following relationship:

$$1.0 < KG/KR < 1.15.$$

**3.** The image display apparatus of Claim 1 or 2, comprising:

a color tone inputting means for inputting a desired color tone,

wherein the control section generates the image data to be displayed by assigning the data of the monochrome image to R, G, and B according to the color tone inputted by the color tone inputting means.

**4.** The image display apparatus of Claim 1 or 2, comprising:

an observer identification code inputting means for inputting an observer identification code which can identify an observer; and
a storage section storing the observer identification code and a color tone desired by each observer with associated with each other,

wherein the control section generates the image data to be displayed by assigning the data of the monochrome image to R, G, and B according to a color tone desired by an observer who is associated with the observer identification code inputted by the observer identification code inputting means.

**5.** An image display method for displaying a monochrome image on a color display section based on an image data to be displayed generated by assigning data of the monochrome image to R, G, and B, comprising:

generating the image data to be displayed by assigning the data of the monochrome image to R, G, and B so as to satisfy a relationship of following formulas (1) and (2) within at least a range between a maximum luminance L0 of an image to be displayed and a luminance L1 which is one thirtieth of the luminance L0:

$$\text{Data-R} = KR \cdot \text{data-B} \quad (0.5 < KR \leq 1) \qquad \text{Formula (1)}$$

$$\text{Data-G} = KG \cdot \text{data-B} \quad (0.5 < KG \leq 1) \qquad \text{Formula (2)}$$

where coefficients KR and KG in the formulas satisfy a following condition of a following formula (3):

$$0.85 < KG/KR < 1.2. \qquad \text{Formula (3)}$$

**6.** The image display method of Claim 5,
wherein the coefficients KR and KG satisfy a following relationship:

$$1.0 < KG/KR < 1.15.$$

**7.** The image display method of Claim 5 or 6,
wherein when a desired color tone is inputted, the image data to be displayed is generated by assigning the data of the monochrome image to R, G, and B according to the inputted color tone.

**8.** The image display method of Claim 5 or 6,

14

wherein when an observer identification code which can identify the observer is inputted, the image data to be displayed is generated by assigning the data of the monochrome image to R, G, and B according to a desired color tone associated with each observer.

**9.** An image displaying program which causes a computer to carry out the method of any one of Claims 5 to 8.

# FIG. 1

# FIG. 2

EP 1 974 660 A1

FIG. 3

## FIG. 4

## FIG. 5

| RGB DATA | FRAME DATA | | | |
|---|---|---|---|---|
| | SECOND FM10a | SECOND FM10b | SECOND FM10c | SECOND FM10d |
| 0 | 0 | 0 | 0 | 0 |
| 1 | 1 | 0 | 0 | 0 |
| 2 | 1 | 0 | 1 | 0 |
| 3 | 1 | 0 | 1 | 1 |
| 4 | 1 | 1 | 1 | 1 |
| 5 | 2 | 1 | 1 | 1 |
| 6 | 2 | 1 | 2 | 1 |
| 7 | 2 | 1 | 2 | 2 |
| 8 | 2 | 2 | 2 | 2 |
| 9 | 3 | 2 | 2 | 2 |
| 10 | 3 | 2 | 3 | 2 |
| 11 | 3 | 2 | 3 | 3 |
| 12 | 3 | 3 | 3 | 3 |
| ⋮ | ⋮ | ⋮ | ⋮ | ⋮ |
| 508 | 127 | 127 | 127 | 127 |
| 509 | 128 | 127 | 127 | 127 |
| 510 | 128 | 127 | 128 | 127 |
| 511 | 128 | 127 | 128 | 128 |
| 512 | 128 | 128 | 128 | 128 |
| ⋮ | ⋮ | ⋮ | ⋮ | ⋮ |
| 1019 | 255 | 254 | 255 | 255 |
| 1020 | 255 | 255 | 255 | 255 |
| 1021 | 255 | 255 | 255 | 255 |
| 1022 | 255 | 255 | 255 | 255 |
| 1023 | 255 | 255 | 255 | 255 |

# FIG. 6

|  | SECOND<br>FM10a DATA | SECOND<br>FM10b DATA | SECOND<br>FM10c DATA | SECOND<br>FM10d DATA |
|---|---|---|---|---|
| RGB DATA: 509 | 128 | 127 | 127 | 127 |
| RGB DATA: 510 | 128 | 127 | 128 | 127 |
| RGB DATA: 511 | 128 | 127 | 128 | 128 |
| RGB DATA: 512 | 128 | 128 | 128 | 128 |

FRAME
CYCLE

RGB DATA DISPLAY CYCLE

# FIG. 7

# FIG. 8

23
OBSERVER IDENTIFICATION CODE INPUTTING SIGNAL

24
STORAGE SECTION

4
BACKLIGHT

2
LIQUID CRYSTAL PANEL

IMAGE SUPPLY APPARATUS
7

INTERFACE
6

CONTROL SECTION
5

LIQUID CRYSTAL DRIVE SECTION
3

EP 1 974 660 A1

<table>
<tr><td colspan="2" align="center"><b>INTERNATIONAL SEARCH REPORT</b></td><td>International application No.<br>PCT/JP2007/050216</td></tr>
</table>

**A. CLASSIFICATION OF SUBJECT MATTER**
*A61B5/00*(2006.01)i, *A61B6/00*(2006.01)i, *G09G3/20*(2006.01)i, *G09G3/36*(2006.01)i, *G09G5/00*(2006.01)i, *G09G5/02*(2006.01)i

According to International Patent Classification (IPC) or to both national classification and IPC

**B. FIELDS SEARCHED**

Minimum documentation searched (classification system followed by classification symbols)
A61B5/00, A61B6/00, G09G3/20, G09G3/36, G09G5/00, G09G5/02

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho    1971-2007   Toroku Jitsuyo Shinan Koho   1994-2007

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2000-330530 A (Fuji Photo Film Co., Ltd.), 30 November, 2000 (30.11.00), Claims; Par. Nos. [0026] to [0065]; Fig. 5 | 1-9 |
| Y | JP 2002-236285 A (Agfa-Gevaert N.V.), 23 August, 2002 (23.08.02), Par. Nos. [0042], [0066]; Fig. 7 | 1-9 |
| A | JP 2003-295851 A (Fuji Photo Film Co., Ltd.), 15 October, 2003 (15.10.03), Claims | 1-9 |
| A | JP 2001-34232 A (Fuji Photo Film Co., Ltd.), 09 February, 2001 (09.02.01), Claims; Fig. 4 | 1-9 |

| ☒ | Further documents are listed in the continuation of Box C. | ☒ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>25 January, 2007 (25.01.07) | Date of mailing of the international search report<br>06 February, 2007 (06.02.07) |
|---|---|
| Name and mailing address of the ISA/<br>Japanese Patent Office | Authorized officer |
| Facsimile No. | Telephone No. |

Form PCT/ISA/210 (second sheet) (April 2005)

22

**INTERNATIONAL SEARCH REPORT**

| International application No. |
| --- |
| PCT/JP2007/050216 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2002-325735 A  (Konica Corp.),<br>12 November, 2002 (12.11.02),<br>Par. Nos. [0075] to [0079] | 1-9 |
| A | JP 2003-265416 A  (Konica Corp.),<br>24 September, 2003 (24.09.03),<br>Par. Nos. [0063] to [0074]; Figs. 5 to 6 | 1-9 |

Form PCT/ISA/210 (continuation of second sheet) (April 2005)

**INTERNATIONAL SEARCH REPORT**
Information on patent family members

International application No.

PCT/JP2007/050216

| JP 2000-330530 A | 2000.11.30 | JP 3853105 B2 | 2006.12.06 |
| | | US 6714208 B1 | 2004.03.30 |
| JP 2002-236285 A | 2002.08.23 | EP 1209511 A1 | 2002.05.29 |
| | | US 6646700 B2 | 2003.11.11 |
| | | US 2002/0063821 A1 | 2002.05.30 |
| JP 2003-295851 A | 2003.10.15 | (Family: none) | |
| JP 2001-34232 A | 2001.02.09 | (Family: none) | |
| JP 2002-325735 A | 2002.11.12 | (Family: none) | |
| JP 2003-265416 A | 2003.09.24 | (Family: none) | |

Form PCT/ISA/210 (patent family annex) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000330530 A
- JP 2001034232 A